# EUROPÄISCHE PATENTANMELDUNG

(11) **EP 2 910 495 A2**
(43) Veröffentlichungstag der Anmeldung: **26.08.2015**
(21) Anmeldenummer: 15151620.0
(22) Anmeldetag: 19.01.2015
(51) Int. Cl.: B65F 1/00

(54) **Folienbeutel und Verfahren zum Herstellen eines Folienbeutels**

(30) Priorität: 20.02.2014 DE 102014102150
(71) Anmelder: Melitta Europa GmbH & Co. KG, 32427 Minden (DE)
(72) Erfinder: Vorfeld, Dr. Udo, 32051 Herford (DE)
(74) Vertreter: Dantz, Jan Henning

(57) **Zusammenfassung**

Ein Folienbeutel (1), insbesondere Müllbeutel, umfasst Beutelwände (2) aus einem ein- oder mehrlagigen Folienmaterial, die an der Innenseite zumindest bereichsweise mit einem geruchsabsorbierenden Wirkstoff versehen sind, wobei der geruchsabsorbierende Wirkstoff zumindest bereichsweise auf die Innenseite einer Beutelwand (2) aufgesprüht ist. Ferner betrifft die Erfindung ein Verfahren zum Herstellen eines Folienbeutels (1).

## Beschreibung

Die vorliegende Erfindung betrifft einen Folienbeutel, insbesondere einen Müllbeutel, mit Beutelwänden aus einem ein- oder mehrlagigen Folienmaterial, die an der Innenseite zumindest bereichsweise mit einem geruchsabsorbierenden Wirkstoff versehen sind, sowie ein Verfahren zum Herstellen eines Folienbeutels.

Die geruchsabsorbierende Wirkung kann schlecht unmittelbar erfasst werden. Daher wird zur Messung der Geruchsabsorption meist über die DIN EN 13725:2003 "Luftqualität - Bestimmung der Geruchskonzentration mittels dynamischer Olfaktometrie" verwendet.

Die EP 1 657 187 offenbart einen Müllbeutel, bei dem an einer Trägerschicht Aktivkohle zur Geruchsabsorption vorgesehen ist. Die Trägerschicht wird mit Aktivkohle belegt und dann auf eine Beutelwand geklebt. Die Herstellung einer solchen Trägerschicht mit Aktivkohle und die spätere Verklebung an dem Folienbeutel sind vergleichsweise aufwändig. Um den Materialeinsatz gering zu halten, werden meist nur kleine Abschnitte des Folienbeutels mit der Trägerschicht versehen.

Die DE 295 05 843 offenbart einen Folienbeutel, bei dem Aromastoffe in das Folienmaterial eingearbeitet sind. Bei solchen Folien besteht das Problem, dass nur wenige Aromastoffe eingesetzt werden können, die den hohen Temperaturen bei der Herstellung des Folienmaterials standhalten.

Es ist daher Aufgabe der vorliegenden Erfindung, einen Folienbeutel und ein Verfahren zu dessen Herstellung zu schaffen, der mit einfachen Mitteln eine geruchsabsorbierende Wirkung bereitstellt.

Diese Aufgabe wird mit einem Folienbeutel mit den Merkmalen des Anspruches 1 und einem Verfahren zum Herstellen eines Folienbeutels mit den Merkmalen des Anspruches 6 gelöst.

Erfindungsgemäß umfasst der Folienbeutel an einer Innenseite einer Beutelwand einen geruchsabsorbierenden Wirkstoff, der zumindest bereichsweise auf die Innenseite der Beutelwand aufgesprüht ist. Dadurch kann der geruchsabsorbierende Wirkstoff ohne Trägermaterial auf die Innenseite der Beutelwand aufgebracht werden, was eine einfache Herstellung ermöglicht. Zudem können auch geruchsabsorbierende Wirkstoffe eingesetzt werden, die den hohen Herstellungstemperaturen für das Folienmaterial nicht standhalten würden, da der Auftrag erst nach der Herstellung des Folienmaterials erfolgt.

Das Aufsprühen des geruchsabsorbierenden Wirkstoffes auf das Folienmaterial kann vollflächig erfolgen, vorzugsweise werden aber ein oder mehrere Streifen des geruchsabsorbierenden Wirkstoffes aufgesprüht. Je nach Art und Zusammensetzung der Sprühlösung und des besprühten Folienmaterials wird der oder die geruchsabsorbierenden Wirkstoffe dabei auf der Beutelwand flächig verteilt. Die Menge an geruchsabsorbierendem Wirkstoff kann in einem Bereich zwischen 3 bis 150 mg, insbesondere 5 bis 100 mg, pro Folienbeutel betragen.

Der geruchsabsorbierende Wirkstoff kann ein einzelner Wirkstoff oder eine Mischung unterschiedlicher Wirkstoffe sein, wobei der Begriff "Wirkstoff' im Sinne der Anmeldung auch Mischungen unterschiedlicher Wirkstoffe enthalten kann. Vorzugsweise enthält der geruchsabsorbierende Wirkstoff ein Zinkricinoleat und/oder ein Saccharomyces Ferment. Auch andere Wirkstoffe können eingesetzt werden.

Bei dem erfindungsgemäßen Herstellungsverfahren wird zunächst eine ein- oder mehrlagige Folie hergestellt, insbesondere extrudiert, und dann mit einem in einem Lösungsmittel gelösten geruchsabsorbierenden Wirkstoff besprüht. Aus der beschichteten Folie wird dann ein Folienbeutel konfektioniert. Das Aufsprühen des gelösten geruchsabsorbierenden Wirkstoffes kann in einem oder mehreren Streifen parallel zur Förderrichtung des Folienmaterials erfolgen, indem eine Dosierpumpe mit entsprechender Sprühdüse vorgesehen wird.

Nach dem Besprühen kann das Lösungsmittel teilweise durch Trocknen entfernt werden. Bei einigen Wirkstoffen ist es allerdings sinnvoll, die Beschichtung mit einer gewissen Feuchtigkeit zu belassen, um ein vollständiges Trocknen zu vermeiden. Denn einige Wirkstoffe haben ohne Wasser eine schlechtere Wirksamkeit als in getrockneter Form. Aufgrund der hohen Wirksamkeit der Wirkstoffe werden auch nur sehr kleine Mengen der Wirkstofflösungen aufgesprüht.

Vorzugsweise wird als Lösungsmittel Wasser und/oder Alkohol, beispielsweise Ethanol, eingesetzt. Das Lösungsmittel kann zusätzlich ein oder auch mehrere Tenside enthalten, um das Anhaften und die Verteilung auf dem Folienmaterial zu verbessern.

Ferner kann dem Lösungsmittel ein Feuchthaltemittel sowie ein Verdickungsmittel zugesetzt sein, um die Funktion der Wirkstofflösung zu verbessern.

Neben den schon genannten Stoffen kann das Lösungsmittel auch noch Duftstoffe und Parfüme enthalten, die die Funktion des geruchsabsorbierenden Wirkstoffes nicht beeinflussen. Es ist aber auch möglich, vollständig auf Duftstoffe zu verzichten.

Der Anteil an Lösungsmittel kann dabei mindestens 20 Gew.-%, insbesondere mehr als 30 Gew.-%, bezogen auf die aufgesprühte Flüssigkeit betragen.

Die Erfindung wird nachfolgend anhand eines Ausführungsbeispiels mit Bezug auf die beigefügte Zeichnung näher erläutert. Es zeigt:

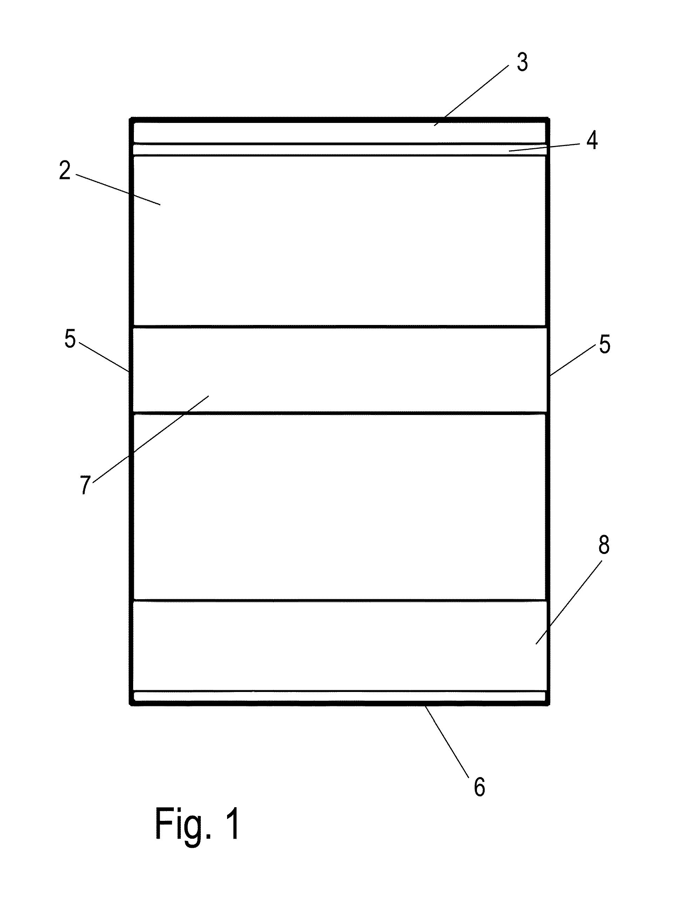

- Figur 1: eine schematische Ansicht einer Beutelwand eines erfindungsgemäßen Folienbeutels.

Ein Folienbeutel 1 umfasst eine Beutelwand 2 aus einem ein- oder mehrlagigen Folienmaterial. Der Folienbeutel 1 besitzt eine Öffnung 3, an der ein Verschlussmittel 4 in Form eines Zugbandes oder Verschlussbandes vorgesehen ist. Der Folienbeutel 1 besitzt zwei Seitennähte 5 und eine Bodennaht 6, die durch Schweißen hergestellt sind, aber auch durch eine Faltkante ersetzt werden können.

An der Innenseite der Beutelwand 2 sind zwei Streifen 7 und 8 vorgesehen, an denen ein geruchsabsorbierender Wirkstoff beschichtet ist. Dabei können die Streifen 7 und 8 unterschiedliche geruchsabsorbierende Wirkstoffe enthalten oder die gleichen Wirkstoffe. Vorzugsweise werden Mischungen verschiedener geruchsabsorbierender Wirkstoffe eingesetzt, insbesondere mit einem Zinkricinoleat und/oder einen Saccharomyces Ferment.

Zur Herstellung des Folienbeutels 1 wird zunächst ein Folienmaterial extrudiert und dann mit einer Lösung mit einem geruchsabsorbierenden Wirkstoff besprüht, so dass das Folienmaterial einen oder mehrere Streifen mit einer Beschichtung aus dem geruchsabsorbierenden Wirkstoff aufweist. Direkt nach dem Aufsprühen kann der Anteil von Wasser und/oder Ethanol in der Sprühlösung mehr als 90Gew.-% betragen. Durch den Einsatz eines Feuchthaltemittels wird die Restfeuchte hoch gehalten, um die Wirksamkeit einiger geruchsabsorbierender Wirkstoffe zu erhalten.

Das Folienmaterial wird danach zu einem Folienbeutel konfektioniert, wobei entsprechende Faltungen, Schweißnähte und andere erforderliche Maßnahmen vorgenommen werden, so dass die mit dem geruchsabsorbierenden Material beschichteten Streifen 7 und 8 an der Innenseite einer Beutelwand angeordnet werden.

In dem dargestellten Ausführungsbeispiel sind zwei Streifen 7 und 8 mit der aufgesprühten Beschichtung vorgesehen. Es ist auch möglich, die Innenseite einer Beutelwand 2 vollflächig zu beschichten oder nur im Bereich benachbart zu der Öffnung 3, an der auch unangenehme Gerüche austreten können.

### Bezugszeichenliste

- 1: Folienbeutel
- 2: Beutelwand
- 3: Öffnung
- 4: Verschlussmittel
- 5: Seitennähte
- 6: Bodennaht
- 7: Streifen
- 8: Streifen

## Patentansprüche

1. Folienbeutel (1), insbesondere Müllbeutel, mit Beutelwänden (2) aus einem ein- oder mehrlagigen Folienmaterial, die an der Innenseite zumindest bereichsweise mit einem geruchsabsorbierenden Wirkstoff versehen sind, **dadurch gekennzeichnet, dass** der geruchsabsorbierende Wirkstoff zumindest bereichsweise auf die Innenseite einer Beutelwand (2) aufgesprüht ist.

2. Folienbeutel nach Anspruch 1, **dadurch gekennzeichnet, dass** ein oder mehrere Streifen (7, 8) mit dem geruchsabsorbierenden Wirkstoff auf der Innenseite der Beutelwand (2) vorgesehen sind.

3. Folienbeutel nach Anspruch 1 oder 2, **dadurch gekennzeichnet, dass** die aufgetragene Menge an dem geruchsabsorbierenden Wirkstoff zwischen 3 bis 150 mg, insbesondere 5 bis 100 mg, pro Folienbeutel (1) beträft.

4. Folienbeutel nach einem der vorhergehenden Ansprüche, **dadurch gekennzeichnet, dass** der geruchsabsorbierende Wirkstoff ein Zinkricinoleat enthält.

5. Folienbeutel nach einem der vorhergehenden Ansprüche, **dadurch gekennzeichnet, dass** der geruchsabsorbierende Wirkstoff ein Saccharomyces Ferment enthält.

6. Verfahren zum Herstellen eines Folienbeutels (1), insbesondere eines Müllbeutels, mit den folgenden Schritten:
- Extrudieren einer Folie;
- Besprühen der Folie mit einem in einem Lösungsmittel gelösten geruchsabsorbierenden Wirkstoff; und
- Konfektionieren der beschichteten Folie zu einem Folienbeutel (1).

7. Verfahren nach Anspruch 6, **dadurch gekennzeichnet, dass** das Lösungsmittel Wasser und/oder Alkohol enthält.

8. Verfahren nach Anspruch 6 oder 7, **dadurch gekennzeichnet, dass** das Lösungsmittel ein oder mehrere Tenside enthält.

9. Verfahren nach einem der Ansprüche 6 bis 8, **dadurch gekennzeichnet, dass** das Lösungsmittel ein Feuchthaltemittel enthält.

10. Verfahren nach einem der Ansprüche 6 bis 9, **dadurch gekennzeichnet, dass** das Lösungsmittel ein Verdickungsmittel enthält.

11. Verfahren nach einem der Ansprüche 6 bis 10, **dadurch gekennzeichnet, dass** das Lösungsmittel ein Duftstoff oder Parfüm enthält.

12. Verfahren nach einem der Ansprüche 6 bis 11, **dadurch gekennzeichnet, dass** der Anteil des Lösungsmittels mindestens 20 Gew.-%, insbesondere mehr als 30 Gew.-%, der aufgesprühten Flüssigkeit ist.
